Europäisches Patentamt

European Patent Office

Office européen des brevets

Publication number: **0 300 630**
**A2**

# EUROPEAN PATENT APPLICATION

(21) Application number: 88306019.6

(51) Int. Cl.⁴: **A61K 31/40**

(22) Date of filing: 01.07.88

| | |
|---|---|
| The title of the invention has been amended (Guidelines for Examination in the EPO, A-III, 7.3). | (71) Applicant: **Regulin Limited** **Level 12 222 Kingsway** **South Melbourne Victoria(AU)** |
| (30) Priority: 03.07.87 AU 2902/87 | (72) Inventor: **Staples, Linton Drew** **103 Normanby Road** **East Kew, AU-3102 Victoria(AU)** |
| (43) Date of publication of application: **25.01.89 Bulletin 89/04** | (74) Representative: **Field, Roger Norton et al** **Brewer & Son Quality House 5-9, Quality** |
| (84) Designated Contracting States: **AT BE CH DE ES FR GB GR IT LI LU NL SE** | **Court Chancery Lane** **London WC2A 1HT(GB)** |

(54) **Melatonin for regulating animal reproduction.**

(57) The present invention relates to a method of regulating the seasonal breeding activity and/or seasonal physiological responses of animals utilising veterinary implants.

Previously described coated veterinary implants allow an active ingredient to be released at a predetermined rate to maintain a predetermined blood level of melatonin.

However, the manipulation of breeding activity was limited to relative brief delays in the onset of puberty or oestrus or relatively brief advancement in the onset of puberty or oestrus.

Accordingly difficulties of the prior art are substantially overcome by using the method of the invention. The method includes subjecting the animal to be treated to a first treatment with a veterinary composition including

(a) an effective amount of an active ingredient selected from melatonin or related chemicals of the indole class, as hereinbefore defined, or mixtures thereof, and

(b) a veterinarily acceptable carrier or excipient therefor;     at a preselected time relative to the normal breeding cycle of the animal;

and subjecting the animal to be treated to at least a second treatment with a veterinary composition as described above at a later preselected time relative to the normal breeding cycle of the animal. A composition for use in the above method is also provided.

EP 0 300 630 A2

## METHOD FOR REGULATING ANIMAL REPRODUCTION

The present invention relates to a method of regulating the seasonal breeding activity and or seasonal physiological responses of animals utilising veterinary implants.

In our earlier Australian Patent Application No. 72915/87 there is described a coated veterinary implant including a veterinary implant including an effective amount of melatonin or related indoles and a veterinarily acceptable carrier or excipient together with a coating which allows the active ingredient to be released at a predetermined rate to maintain a predetermined blood level of melatonin.

Whilst the coated veterinary implant described above was found to be useful in regulating seasonal breeding activity and/or seasonal physiological responses via implantation of the animals to be treated. the manipulation of breeding activity was limited to relatively brief delays in the onset of puberty or oestrus or relatively brief advancement in the onset of puberty or oestrus.

It is accordingly an object of the present invention to overcome, or at least alleviate. one or more of the difficulties related to the prior art.

Accordingly in a first aspect there is provided a method of regulating the seasonal breeding activity and/or seasonal physiological responses of animals which method includes subjecting the animal to be treated to a first treatment with a veterinary composition including

(a) an effective amount of an active ingredient selected from melatonin or related chemicals of the indole class, as hereinbefore defined, or mixtures thereof, and

(b) a veterinarily acceptable carrier or excipient therefor;    at a preselected time relative to the normal breeding cycle of the animal;

and subjecting the animal to be treated to at least a second treatment with a veterinary composition as described above at a later preselected time relative to the normal breeding cycle of the animal.

The first and subsequent veterinary treatments in use may release the active ingredient at a rate sufficient to maintain the blood level of melatonin or related chemicals of the indole class at or above a level sufficient. and for a period sufficient, to regulate seasonal breeding activity and/or seasonal physiological responses of an animal to be treated.

In a preferred aspect of the present invention the seasonal breeding activity is regulated by delaying the onset of the normal breeding cycle; and wherein the veterinary composition is administered to the animal prior to the end of the previous normal breeding cycle.

It would be understood that administration prior to the end of the previous cycle will delay the end of that cycle and in turn delay the onset of the next cycle.

In a further preferred aspect of the present invention the seasonal breeding activity and/or seasonal physiological responses are regulated by advancing the onset of the normal breeding cycle wherein the first veterinary treatment is administered within a predetermined time frame between the end of the previous normal breeding cycle and the beginning of the next normal breeding cycle within which the animal is receptive to treatment with melatonin or related chemicals of the indole class as hereafter defined;

and the second veterinary treatment is subsequently administered to the animal within a predetermined time frame dictated by the first veterinary treatment within which the animal is again susceptible to treatment with melatonin or related chemicals of the indole class as hereinafter defined.

The active ingredient of the veterinary composition according to the present invention includes melatonin and related chemicals of the indole class. which term, as used herein in the specification and claims refers to

MELATONIN (N-acetyl 5-methoxy tryptamine)
MELATONIN analogues substituted at the amino nitrogen, e.g:
N-propionyl/N-butyryl
MELATONIN analogues substituted at the 5- position,
e.g.: 5-ethoxy (i.e. N-acetyl O-ethyl serotonin)
5-propoxy (i.e. N-acetyl O-propyl serotonin)
MELATONIN analogues substituted at the 6- position,
e.g.: Halo-substituted: 6-fluoro/6-bromo/6-chloro/6-iodo
Hydroxy- substituted
Suphatoxy- substituted
Alkyl- substituted: 6-methyl/6-ethyl/6-isopropyl etc.
Alkoxy- substituted, e.g. 6-methoxy
Silylated, e.g. 6-(t-butyl)dimethylsiloxy
SATURATED MELATONIN DERIVATIVES. such as (2.3-dihydro)melatonin and 6-chloro-(2.3-dihydro)-

2

melatonin (together with all the 5-, 6- and N- substituted saturated derivatives thereof as described above

RING-CLEAVED MELATONIN derivatives (including all the 5- 6- and N- substituted derivatives thereof as described above, and similarly, saturated derivatives thereof), such as

N-acetyl 5-hydroxy kynurenamine

N-acetyl 5-methoxy kynurenamine

RELATED INDOL (SEROTONIN) derivatives, such as

N-acetyl serotonin

N,O-diacetyl serotonin

N-acetyl tryptamine

Serotonin

5-methoxyl tryptophol

5-methoxy tryptamine

OTHER DERIVATIVES and RELATIVES of MELATONIN, such as

N-alkyl and/or N,N-dialkyl analogues of tryptamine, serotonin and melatonin

N-aryl and/or N,N-diaryl analogues of tryptamine, serotonin and melatonin

Similarly, mixed N-alkyl, N-aryl derivatives 6-substituted derivatives of tryptamine, Ureido- analogues of tryptamine and serotonin; or mixtures thereof.

Accordingly the term melatonin or like treatment as used herein in the description and claims refers to treatment with any of the abovementioned compounds.

The active ingredient may be present in an amount of from approximately 25 to 98% by weight based on the total weight of the veterinary composition.

The veterinary composition may be provided in a unit dosage form. The veterinary composition may be provided in a sustained release form. The veterinary composition may be provided as a veterinary implant or series of veterinary implants. The veterinary implant may be a coated veterinary implant including an effective amount of an active ingredient selected from melatonin or related chemicals of the indole class, as hereinafter defined, or mixtures thereof;

a veterinarilly acceptable carrier or excipiant thereof; and

a coating for said veterinary implant formed from a physiologically compatible polymeric coating composition.

More preferably the coated veterinary implant includes

an implant core including

from approximately 1 to 75% by weight based on the total weight of the coated implant of a water insoluble cellulose compound

from approximately 75 to 98% by weight based on the total weight of the coated implant of melatonin; and

an implant coating including

from approximately 0.01% to 2.0% by weight based on the total weight of the coated implant of a film-forming high molecular weight polymer.

The veterinarily acceptable carrier or excipient may include a compressible pharmaceutical vehicle selected to control the release rate of the active ingredient. The carrier or excipient may be present in amounts of approximately 1 to 75% by weight based on the total weight of the coated implant.

Such a vehicle may be a high molecular weight form of ethyl cellulose. The form of ethyl cellulose may be chosen from the range of ethoxylated cellulose derivatives sold under the trade designation "Ethocel" and available from the Dow Chemical Company. Ethocel Standard and Ethocel Medium, both of viscosity grades between 7 cP and 100 cP, are preferred.

Alternatively, or in addition, the veterinary carrier or excipient may include an acid salt. The acid salt may be a phosphate salt. The vehicle may be an alkaline earth metal salt. A calcium phosphate is preferred. A hydrated acid salt may be used. A dibasic calcium phosphate dihydrate is preferred. The acid salt may be a calcium phosphate of the type sold under the trade designation "Encompress" and available from Edward Mendell Co. Inc., New York, United States of America.

Preferably, the veterinary implant (a) according to the present invention is formed by granulation and compression, and may therefore further include an effective amount of

(iii) a granulating agent, and/or

(iv) the compression lubricant.

The granulating agent (iii) may be selected from a high molecular weight compound or a cellulose compound or mixtures thereof. The high molecular weight compound or the cellulose compound may be a water-insoluble compound. As the high molecular weight compound, vinyl polymer may be used.

Polyvinylpyrrolidone (PVP) is preferred. The PVP utilised in the implants according to this aspect of the present invention may be selected from the range of vinyl pyrrolidione polymers manufactured and supplied by the CAF Corporation of the United States of America under the trade designation "Plasdone", including Plasdone K-29/32 and Pladone K-90. Plasdone K-29/32 has a volume average molecular weight of approximately 38,000 while the K-90 form has a volume average molecular weight of approximately 630,000.

The molecular weight of the polymer affects the following properties of the material.

(1) Viscosity in solution.

(2) Adhesive properties.

(3) Rate of solution (dissolution).

(4) Rate of absorption and excretion.

As the K-value of Plasdone excipient increases, values for the first two properties listed above increase while the last two decrease.

The cellulose compound may be selected from ethyl cellulose, methyl cellulose, and cellulose esters such as cellulose acetate, cellulose acetate phthalate or compounds sold under the trade designation "Methocel" and "Ethocel" and available from Dow Chemical Company. Alternatively, or in addition, naturally occurring, water-insoluble high molecular weight compounds such as the waxes, for example, beeswax, may be included.

Regardless of which granulating agent (iii) is chosen, that agent may be present in amounts of from approximately 1% to 10% by weight, preferably 1 to 5% by weight based on the total weight of the veterinary implant.

The compression lubricant (iv) may be present in an amount of approximately 0.1 to 5% by weight, preferably 0.5 to 2.0% by weight, based on the total weight of the coated veterinary implant. The compression lubricant additionally may function as a tabletting binder. The lubricant may be a food grade lubricant. The lubricant may be a food source lubricant. The lubricant may be derived from vegetable oil. The lubricant may be a lubricant of the type sold under the trade designation "Lubritab" and available from Edward Mendell Co. Inc., New York, United States of America.

The physiologically compatible polymeric coating composition of the veterinary implant coating (b) may include a film-forming high molecular weight polymer. The film-forming high molecular weight polymer may be water-insoluble. Such a polymer may be a form of ethyl cellulose. The form of ethyl cellulose may be chosen from the range of ethoxylated cellulose derivatives sold under the trade designation "Ethocel" and available from the Dow Chemical Company. Ethocel standard and Ethocel Medium, both of viscosity grades between 7 cP and 100cP, are preferred. The polymer may be provided in the form of an emulsion, dispersion or solution.

The film-forming high molecular weight polymer may be a form of acrylic polymer. The form of acrylic polymer may be selected from a group of polymers and co-polymers synthesised from acrylic and methacrylic acid, esters thereof and mixtures thereof. The acrylic polymer may be selected to form a water-insoluble film whose permeability is independent of pH. The acrylic polymer may be chosen from the range of coating materials sold under the trade designation "Eudragit" and available from Rohm Pharma GmbH. of Darmstadt, Federal Republic of German. Eudragit RLa and Eudragit RS are preferred.

Alternatively an inorganic film-forming high molecular weight polymer coating may be used. A silicon polymer may be used. A silastic-type polymer coating may be used.

The physiologically compatible polymeric coating composition may further include a diluent or solvent for the film-forming high molecular weight polymer. The diluent or solvent for preparation of the physiologically degradable polymeric coating composition may be chosen so as not to dissolve the active ingredient from the implant during manufacture. Accordingly, the diluent or solvent may be an organic solvent, or a mixture of organic solvents. Where esters of cellulose are chosen as the polymer, the preferred diluent or solvent may be dichloromethane. Alternatively, where acrylic resins are chosen as the coating substance, a mixture of acetone and isopropanol may be the preferred diluent or solvent.

The physiologically compatible polymeric coating composition may further include an effective amount of a plasticising agent. Concentrations of plasticising agent of between 0.5% and 10% by volume of the final solvent may be used. Concentrations in the range 2% to 6% by volume of the final solvent are preferred. The plasticiser may be an organic ester. The dibutyl ester of phthalic acid is preferred. Alternatively the plasticiser may be a glycol. Glycerol or polyethylene glycol may be used.

Preferably the coated veterinary implant includes

(a) a veterinary implant including
approximately 2.5 to 100 milligrams more preferably 17.5 to 30 millograms of melatonin,

0 to approximately 0.2 milligrams of polyvinylpyrrolidone;

approximately 2.5 to 5 milligrams of ethyl cellulose; and

approximately 0.2 to 0.3 milligrams of a compression lubricant, and

(b) a first coating for said veterinary implant including approximately 0.01 to 1.0 milligrams of ethyl cellulose polymer.

Preferably the coated veterinary implant further includes

(c) a second coating for said veterinary implant including apprpximately 0.01 to 1.0 milligrams of ethyl cellulose polymer.

Preferably, the coated veterinary implant is of generally cylindrical shape and having dimensions of approximately 2 to 10mm, preferably 2 to 5mm long and 2 to 3mm preferably 2.5mm in diameter.

We have established that the time frame within which the animal is receptive to melatinin or like treatment will vary with species of animal, breed of animal and geographical latitude. However, the first time frame may occur approximately 2 to 12 weeks prior to the onset of the next normal breeding cycle.

A subsequent time frame within which the animal is again receiptive to melatonin or like treatment may occur approximately 2 to 12 weeks after the peak of the next normal breeding cycle.

Where the animal is a sheep the first time frame occurs between approximately 3 and 8 weeks prior to the onset of the next normal breeding cycle and a subsquent time frame occurs between approximately 3 and 8 weeks after the peak of the next normal breeding cycle.

It has been found that treatment of an animal with a single implant as described in our earlier Australian Patent Application 72915/87 the entire disclosure of which is incorporated herein by reference to advance the onset of the normal breeding cycle, whilst successful, is only successful for advancement of a period of weeks rather than months. For example, treatment with a coated veterinary implant in late spring for animals such as ewes, goats and the like, will be successful. However, such a treatment in early spring has been found to produce no response from such animals.

However, we have now found that the treatment regimen involving at least two melatonin treatments, so described above will be successful in permitting the advance of breeding activity for a considerably longer period than is achievable utilising a single implant.

Whilst we do not wish to be restricted by theory, it is postulated that the response of the animals to the administration of the first veterinary composition is recognised by the animals as the stimulus of a very short or even infinitely short day length. Thus the days surrounding the treatment are recognised by the animal as relatively long. Since it is postulated that the time frame of window within which the animals are receptive to melatonin treatment is generated by the number of prior long days, the window of receptivity is advanced.

In a further preferred aspect of the present invention wherein when the first treatment generates a second peak in the next normal breeding cycle in the animal, a further treatment with a veterinary composition is administered after the first peak in the next normal breeding cycle.

In a still further preferred aspect of the present invention the method of regulating the seasonal breeding activity and/or seasonal physiological responses in animals as described above may be amplified by repeating the treatments on an annual basis.

It has been found that the duplication of treatment over a period of years amplifies the effects. For example the advancement of the onset of the normal breeding cycle may be brought forward to an earlier time each year as required.

It will be understood that the method for regulating the seasonal breeding activity and/or seasonal physiological responses in animals may be undertaken in conjunction with other known regulating techniques. Such techniques may include animal priming including light and dark priming.

The present invention will now be more fully described with reference to the accompanying examples. It should be understood, however, that the description following is illustrative only and should not be taken in any way as a restriction on the generality of the invention described.

For example, the present invention has been described in relation to the manipulation of seasonal breeding activity. The treatments described above are equally applicable to the regulation of other seasonal physiological responses in animals including development of pellage. In particular it has been discovered that the extension of seasonal breeding activity over an extended period also results in a reduction or elimination of seasonal tenderness in the pellage of animals including goats and ewes. Similarly, an increase in fibre growth has been noted with an attendant reduction in the amount of shedding in such animals.

Further the present invention has been described with reference to its effect on short day breeding

5

animals such as goats, sheep, deer, mink and the like. However the method of regulating the seasonal breeding activity and/or seasonal physiological responses may be applied equally to long day breeders such as horses.

## EXAMPLE 1

A flock of Corriedale ewes were treated with coated veterinary implants including melatonin as the active ingredient of the type described in Australian Patent Application No. 72915/87. The coated veterinary implants had the following composition.

| Coated Veterinary Implants - Formulation | | |
|---|---|---|
| Implant Cores | | |
| Ethylcellulose (Ethocel S100) Ethanol 95% Melatonin Hydrogenated Vegetable Oil (Lubritab) Quinoline Yellow | to | 10 g 100 mL 200 g 3 % of sieved granule weight 0.4 g |
| Implant Coating | | |
| Coating Solution 1: | | |
| Ethylcellulose (Ethocel M50) Dibutylphthalate Dichloromethane | (1.25%) (0.5%) to | 1.875 g 0.875 mL 150 mL |
| Coating Solution 2: | | |
| Ethylcellulose (Ethocel M50) Dibutylphthalate Dichloromethane Implant Cores Purified Talc | (0.625%) (0.25%) to | 1.875 g 0.875 mL 300 mL 300 g 1.5 g |

## FIELD TRIALS

## General Experimental Procedure

Trials were conducted on Department of Agriculture and Rural Affairs Research Institutes and on private farms. For farm trials the testing protocol involved pre-visits and discussions with the co-operating farmer. Ewes were individually tagged and weighed then allocated to experimental groups either randomly or where appropriate according to age or weaning period. Trial animals were grazed under normal management conditions for the farm and received normal animal health treatments such as for fly strike (e.g. crutching, jetting) and parasite control (e.g. drenching) throughout the trial period. Treated animals were given no supplementary treatments which would in any way influence the assessment of the implant treatment effect.

Implants were inserted by DARA staff and all rams used underwent health checks including examination of feet and mouth, testicle palpation, blood tests for brucellosis and semen tests (mortility and morpholgy of sperm after electroejection).

Ram percentages were normal for breeding flocks, i.e. 2 to 3%. Joining times were selected by the farmer in consultation with DARA staff. Joining times chosen were appropriate for each farmer's requirements but all joinings were early in relation to the peak seasonal reproductive performance for the breed under test.

For farm trials all treatment ewes were usually joined as one group. When separate mating groups were required, such as for "ewe effect" controls on farms or for Institute trials, rams were allocated randomly to experimental groups. All rams were fitted with "Sire Sine" harnesses and crayons. Crayons were changed every fortnight. Ewes were weighed at joining.

Mating flocks were yarded at intervals of 1 to 4 days throughout the joining period and ewes were examined for mating marks. Where possible, cross checks were made of previous mating marks for ewes which mated more than once.

All ewes were weighed at the end of joining and, where appropriate, pregnancy was diagnosed by ultrasonic scanning at mid gestation (approximately 70 days from ram introduction). The number of fetuses present and fetal age was estimated without reference to experimental group. After initial diagnosis, cross reference was made to expected fetal age on the basis of mating records and anomalies (e.g. missed mating marks or breakthrough oestrus in pregnant ewes) were corrected.

Animals from which certain data was not recorded, were excluded from analysis.

In Corriedale ewes malatonin administered via s/c implants (TM "Regulin" Gene Link Australia Ltd) induces an early seasonal rise in proportion of ewes ovulating (PO) and ovulations/ewe ovulating (OR). This response can be exploited if conceptions are timed to coincide with peak ovarian response (2). However, the treatment has now been shown to also cause a transient decline in PO AND ESPECIALLY OR at about 5 months after start of treatment (P $0.05^*$ $-0.001^{***}$, Table 1). This effect may be due, to "refractoriness" to treatment or to a "long day" response at the end of treatment.

TABLE 1

| Long term trends in OR in Corriedale ewes treated with melatonin in Oct-Nov (n = 90-100/group) T @ day 0-24.10.85. | | | | | | | | | | | |
|---|---|---|---|---|---|---|---|---|---|---|---|
| Days | 53 | 74 | 97 | 124 | 162 | 203 | 252 | 273 | 302 | 329 | 358 |
| Control | 1.04 * | 1.08 *_ ** | 1.39 | 1.43 | 1.67 *_ ** | 1.57 ** | 1.37 * | 1.29 | 1.32 | 1.12 | 1.11 |
| Treated | 1.34 | 1.70 | 1.39 | 1.29 | 1.21 | 1.30 | 1.58 | 1.45 | 1.37 | 1.40 | 1.10 |

The results of the various treatment regimens are illustrated in the following figures.

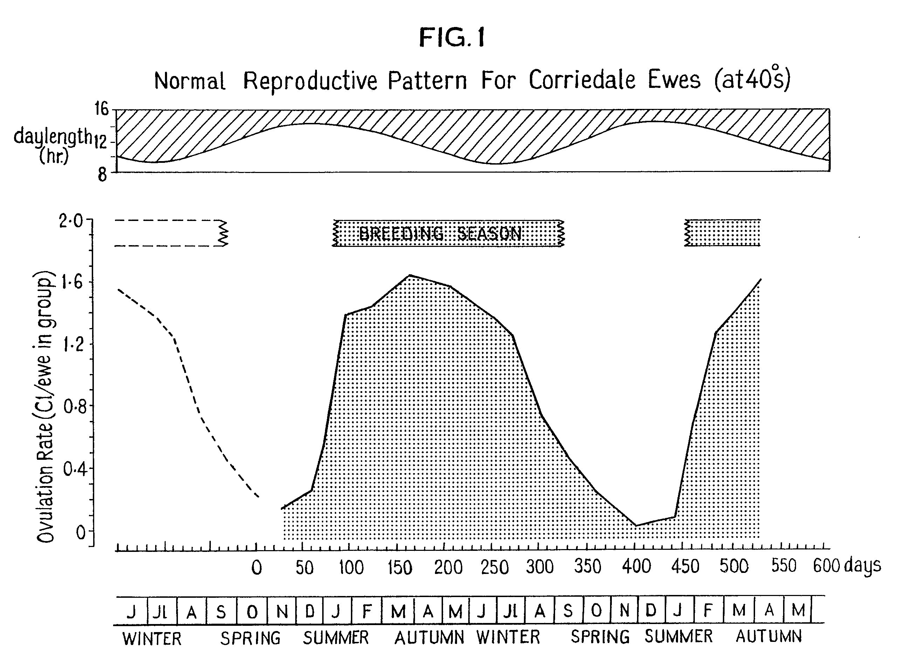

Figure 1 illustrated the normal reproductive pattern for untreated Corriedale ewes.

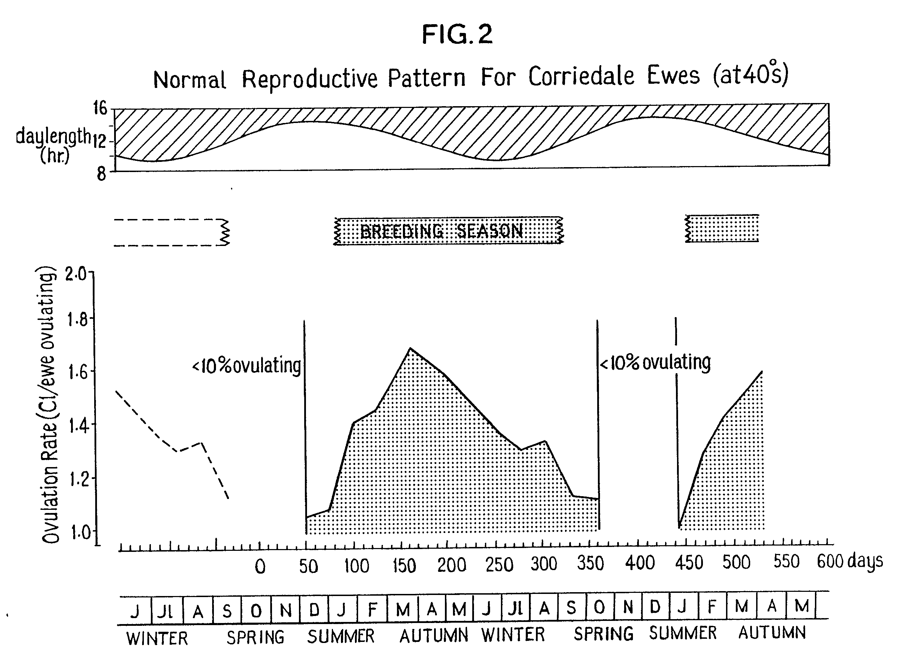

Figure 2 illustrates the normal reproductive pattern showing ovulation rate relative to the number of ewes ovulating.

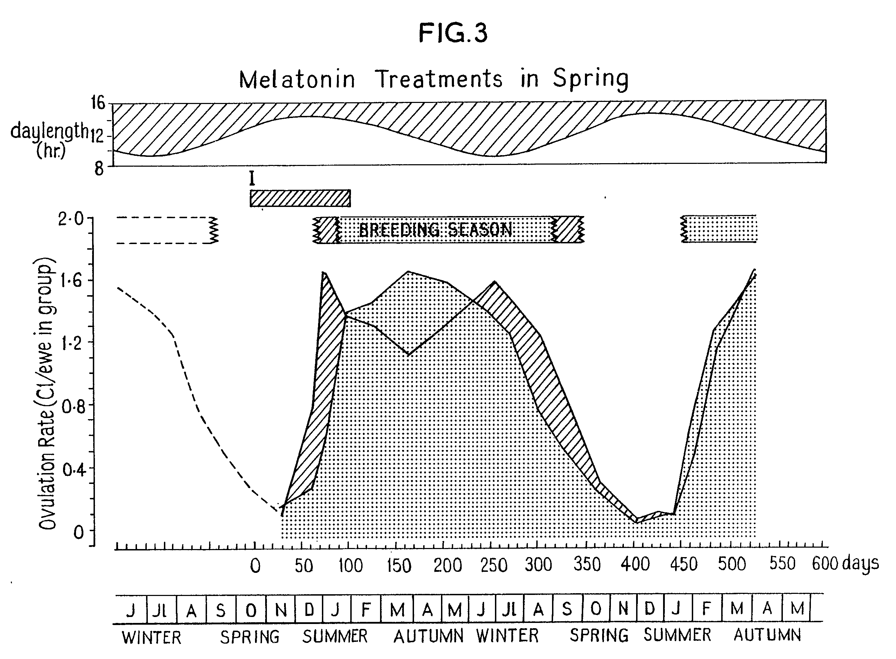

Figure 3 illustrates the extension of breeding season via melatonin implants in spring.

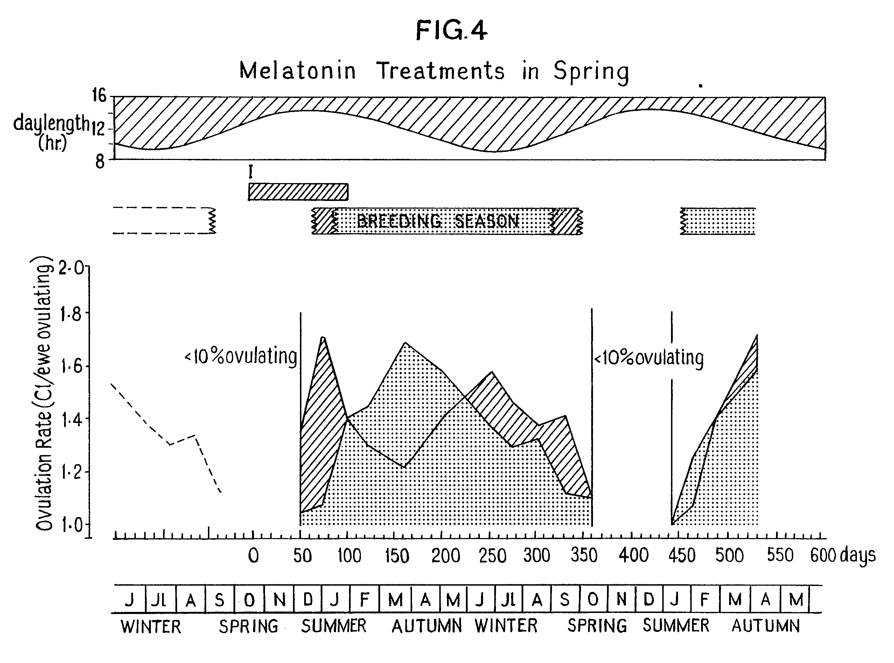

Figure 4 illustrates extension of breeding season relative to the number of ewes ovulating in spring.

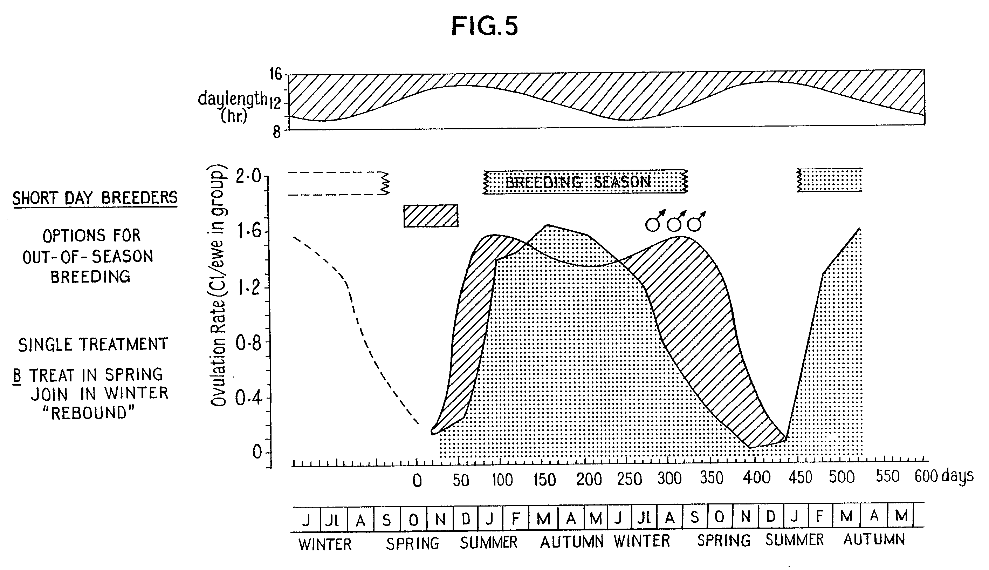

Figure 5 illustrates an implant prior to the onset of the normal breeding cycle and generating a second peak during which joining is undertaken.

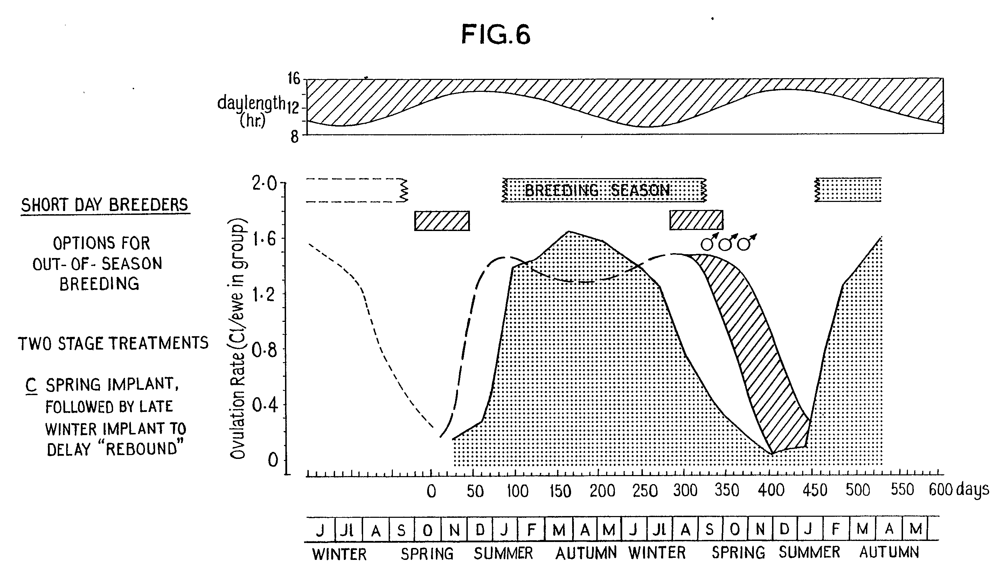

Figure 6 illustrates an implantation prior to the normal breeding cycle followed by a further implantation to extend the second peak.

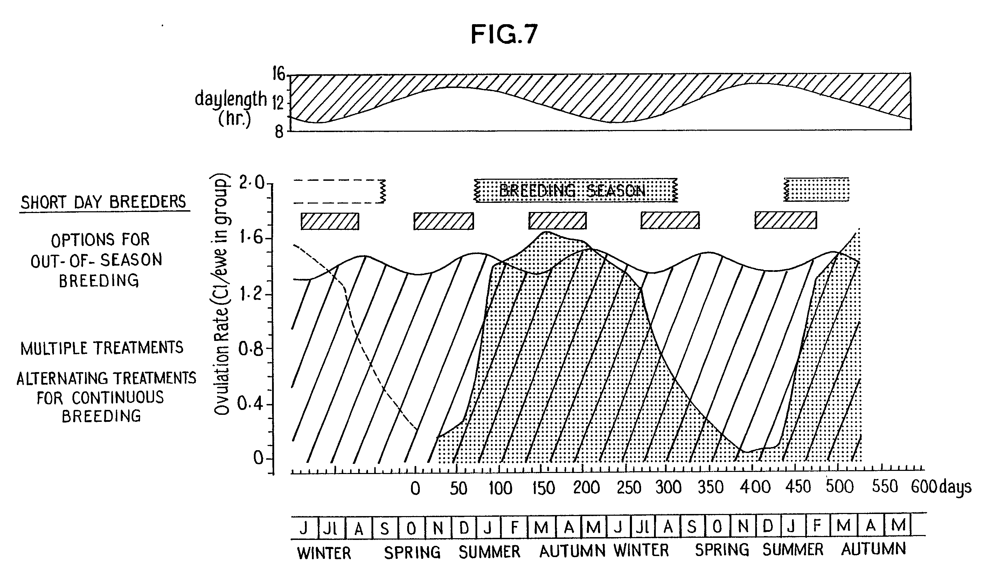

Figure 7 illustrates a series of implantations to provide substantially continuous breeding.


## EXAMPLE 2


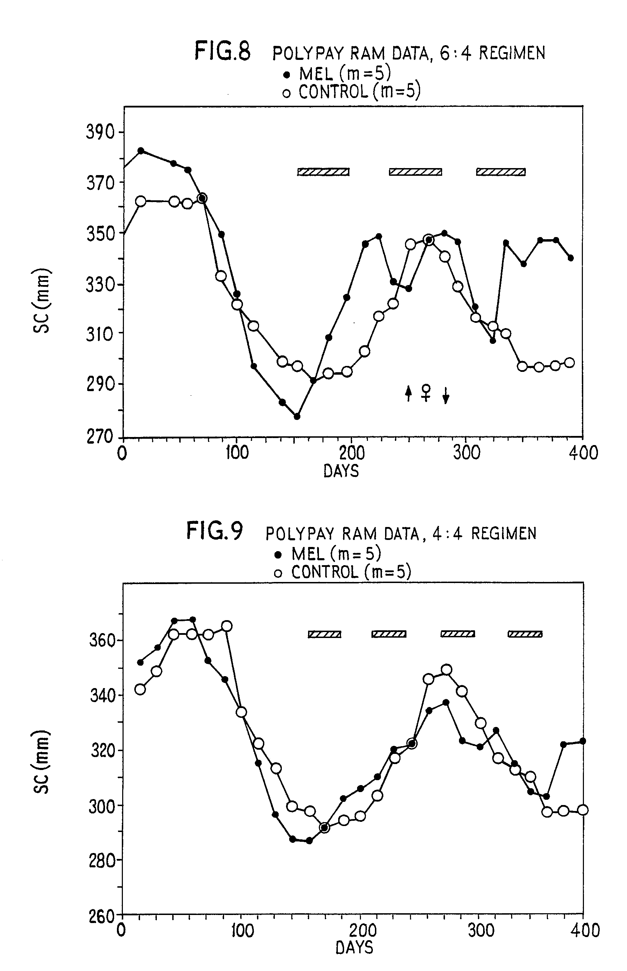

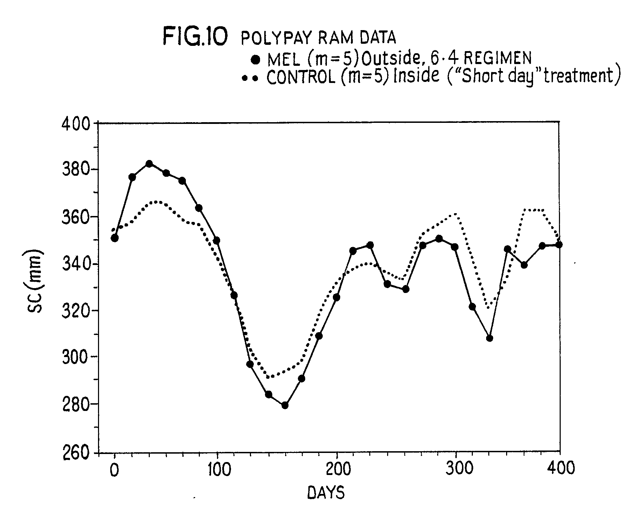

Studies were done to compare the effect of time and duration of melatonin (M) administration on scrotal circumference (SCIR) and fertility of rams used for out-of-season breeding. To gain control of the scrotal cycle, rams were confined in barns on alternating 16 wk periods of long (16L:8D) and short days (8L:16D) for 6-8 months. In study one, mature Booroola crossbred rams (n = 6) were given biodegradable s.c. implants (Gene Link Australia) of M (6wk duration) or left untreated (n = 6) at peak SCIR under short days. Three rams from each treatment were moved to long days. Six weeks later in April, rams were bred to equal numbers of ewes (n = 20) under ambient light conditions. Results showed that blood M concentration measured biweekly at 1000-1400 h was higher (P <.05) in implanted (285±21) compared to nonimplanted (180±33) rams. Fertility (80-100%) did not differ (P>.05) by ram treatment. Average changes in SCIR from peak to nadir were similar (P>.05) across light and M treatment groups. In a second experiment, effects of M given at nadir SCIR under long days were examined. Mature Polypay rams (n = 10) were implanted with M (10 d duration) weekly for 6 (6:4) (Figure 8) or 4 (4:4) (Figure 9) wks. with each implant period followed by 4 wks without the hormone. Scrotal changes and fertility of M implanted rams were also compared with rams (n = 5) moved from long to short days as an alternative method to regulate reproduction (Figure 10). Treatments began 4 months before breeding in April and continued 6 months after breeding ended in May. Results showed that the 6:4 M treatment advanced peak SCIR by 45 days compared with 4:4 or short day treatments. Fertility (82-86%) determined by pregnancy diagnosis of ewes (n = 300) 60-90 d after breeding was similar (P ≥.05) for both M and short day treatments. Although SCIR progressively decreased 15% from peak (349mm) to nadir (297mm) in rams moved from short days to ambient long days in April, M given to rams at a 6:4 sequence resulted in repetitive oscillations in SCIR with mean SCIR differing by only 3% of peak (338mm). In summary, study one indicates that M given for 6 wks to rams when SCIR is maximal under short days does not prevent regression of the testes nor affect fertility. This suggests that rams become refractory to continuous M treatment at this phase of the scrotal cycle. Rams however respond to M when given at nadir SCIR as demonstrated in study two by the stimulatory affect of M on SCIR. Phasic delivery of M (6 wk-on, 4 wk-off) shows promise for regulation of fertility of the ram and may provide an alternative to phoperiodic management in confinement.

Finally, it is to be understood that various other modifications and/or alterations may be made without departing from the spirit of the present invention as outlined herein.

## Claims

1. A method of regulating the seasonal breeding activity and/or seasonal physiological responses of animals which method includes subjecting the animal to be treated to a first treatment with a veterinary composition including

(a) an effective amount of an active ingredient selected from melatonin or related chemicals of the indole class, as hereinbefore defined, or mixtures thereof, and

(b) a veterinarily acceptable carrier or excipient therefor;

at a preselected time relative to the normal breeding cycle of the animal:

and subjecting the animal to be treated to at least a second treatment with a veterinary composition as described above at a later preselected time relative to the normal breeding cycle of the animal.

2. A method according to claim 1 wherein the first and subsequent treatments, in use, release the active ingredient at a rate sufficient to maintain the blood level of melatonin or related chemicals of the indole class at or above a level sufficient, and for a period sufficient, to regulate seasonal breeding activity and/or seasonal physiological responses of an animal to be treated.

3. A method according to claim 2 wherein the seasonal breeding activity and/or seasonal physiological responses are regulated by advancing the onset of the normal breeding cycle wherein the first veterinary treatment is administered within a predetermined time frame between the end of the previous normal breeding cycle and the beginning of the next normal breeding cycle within which the animal is receptive to treatment with melatonin or related chemicals of the indole class as hereinbefore defined;

and the second veterinary treatment is subsequently administered to the animal within a predetermined time frame dictated by the first veterinary treatment within which the animal is again susceptible to treatment with melatonin or related chemicals of the indole class as hereinbefore defined.

4. A method according to claim 3 wherein the time frames within which the animal is receptive to melatonin or like treatment vary with the species of animal, breed of animal and geographical latitude.

5. A method according to claim 4 wherein the first time frame occurs approximately 2 to 12 weeks prior to the onset of the next normal breeding cycle.

6. A method according to claim 5 wherein a subsequent time frame occurs approximately 2 to 12 weeks after the peak of the next normal breeding cycle.

7. A method according to claim 6 wherein the animal is a sheep and wherein the first time frame occurs between approximately 3 and 8 weeks prior to the onset of the next normal breeding cycle and a subsquent time frame occurs between approximately 3 and 8 weeks after the peak of the next normal breeding cycle.

8. A method according to claim 3 wherein when the first treatment generates a second peak in the next normal breeding cycle in the animal, a subsequent treatment with the veterinary composition is administered after the first peak in the next normal breeding cycle.

9. A method according to claim 3 wherein the veterinary composition is provided as a veterinary implant or series of veterinary implants.

10. A method according to claim 9 wherein the veterinary implant or series of veterinary implants includes at least one coated veterinary implant including an effective amount of an active ingredient selected from melatonin or related chemicals of the indole class, as hereinbefore defined, or mixtures thereof;

a veterinarily acceptable carrier or excipient therefor; and

a coating for said veterinary implant formed from a physiologically compatible polymeric coating composition.

11. A method according to claim 10 wherein the at least one coated veterinary implant includes

an implant core including

from approximately 1 to 75% by weight based on the total weight of the coated implant of a water insoluble cellulose compound

from approximately 75 to 98% by weight based on the total weight of the coated implant of melatonin; and

an implant coating including

from approximately 0.01% to 2.0% by weight based on the total weight of the coated implant of a film-forming high molecular weight polymer.

12. A composition comprising (a) an effective amount of an active ingredient selected from melatonin or related chemicals of the indole class, as hereinbefore defined, or mixtures thereof, and (b) a veterinarily acceptable carrier or excipient therefor; for use in the method of any one of the preceding claims.

# FIG.1

## Normal Reproductive Pattern For Corriedale Ewes (at 40°s)

EP 0 300 630 A2

FIG.2

Normal Reproductive Pattern For Corriedale Ewes (at 40°s)

EP 0 300 630 A2

# FIG.3

## Melatonin Treatments in Spring

EP 0 300 630 A2

FIG.4

Melatonin Treatments in Spring

EP 0 300 630 A2

# FIG.5

daylength (hr.)

SHORT DAY BREEDERS

OPTIONS FOR
OUT-OF-SEASON
BREEDING

SINGLE TREATMENT

B TREAT IN SPRING
JOIN IN WINTER
"REBOUND"

Ovulation Rate (CL/ewe in group)

BREEDING SEASON

| J | JL | A | S | O | N | D | J | F | M | A | M | J | JL | A | S | O | N | D | J | F | M | A | M |

WINTER    SPRING    SUMMER    AUTUMN    WINTER    SPRING    SUMMER    AUTUMN

EP 0 300 630 A2

# FIG.6

SHORT DAY BREEDERS

OPTIONS FOR
OUT-OF-SEASON
BREEDING

TWO STAGE TREATMENTS

C SPRING IMPLANT,
FOLLOWED BY LATE
WINTER IMPLANT TO
DELAY "REBOUND"

EP 0 300 630 A2

# FIG.7

SHORT DAY BREEDERS

OPTIONS FOR
OUT-OF-SEASON
BREEDING

MULTIPLE TREATMENTS

ALTERNATING TREATMENTS
FOR CONTINUOUS
BREEDING

daylength (hr.)

Ovulation Rate (Cl/ewe in group)

BREEDING SEASON

| J | Jl | A | S | O | N | D | J | F | M | A | M | J | Jl | A | S | O | N | D | J | F | M | A | M |

WINTER   SPRING   SUMMER   AUTUMN WINTER   SPRING   SUMMER   AUTUMN

EP 0 300 630 A2

FIG.8   POLYPAY RAM DATA, 6:4 REGIMEN
● MEL (m=5)
○ CONTROL (m=5)

FIG.9   POLYPAY RAM DATA, 4:4 REGIMEN
● MEL (m=5)
○ CONTROL (m=5)

FIG.10 POLYPAY RAM DATA
● MEL (m=5) Outside, 6·4 REGIMEN
●● CONTROL (m=5) Inside ("Short day" treatment)